# EUROPEAN PATENT SPECIFICATION

(11) **EP 1 784 633 B1**
(45) Date of publication and mention of the grant of the patent: **09.11.2011**
(21) Application number: 05768147.0
(22) Date of filing: 17.08.2005
(51) Int. Cl.: G01N 21/64

(54) **A METHOD OF ANALYSING A SAMPLE AND APPARATUS THEREFOR**
VERFAHREN ZUR ANALYSE EINER PROBE UND VORRICHTUNG DAFÜR
PROCEDE D'ANALYSE D'UN ECHANTILLON ET APPAREIL ASSOCIE

(30) Priority: 01.09.2004 GB 0419325; 23.06.2005 GB 0512811
(43) Date of publication of application: 16.05.2007
(60) Divisional application: 10182689.9
(73) Proprietor: Perkinelmer Singapore PTE Ltd., Singapore 139947 (SG)
(72) Inventor: BLACKMORE, Colin, PerkinElmer Life Sciences, Buckinghamshire HP9 2FX (GB); COURTNEY, Patrick, PerkinElmer Life Sciences, BuckinghamshireHP9 2FX (GB); FITCH, Alistair, PerkinElmer Life Sciences, Buckinghamshire HP9 2FX (GB); LADHA, Shab, PerkinElmer Life Sciences, Buckinghamshire HP9 2FX (GB)
(74) Representative: Sharrock, Daniel John
(86) International application number: PCT/GB2005/003226
(87) International publication number: WO 2006/024819

(56) References cited:
- EP-A- 1 146 480
- WO-A-00/71028
- WO-A-2004/046691
- US-A- 4 629 687
- US-A- 5 932 872
- US-A1- 2003 026 762
- US-B1- 6 259 524
- WEDEKIND P ET AL: "SCANNING MICROPHOTOLYSIS:A NEW PHOTOBLEACHING TECHNIQUE BASED ON FAST INTENSITY MODULATION OF A SCANNED LASER BEAM AND CONFOCAL IMAGING" JOURNAL OF MICROSCOPY, OXFORD, GB, vol. 176, no. PART 1, October 1994 (1994-10), pages 23-33, XP002901175 ISSN: 0022-2720
- BENSON D M ET AL: "DIGITAL IMAGING FLUORESCENCE MICROSCOPY SPATIAL HETEROGENEITY OF PHOTOBLEACHING RATE CONSTANTS IN INDIVIDUAL CELLS" JOURNAL OF CELL BIOLOGY, vol. 100, no. 4, 1985, pages 1308-1324, XP009055740 ISSN: 0021-9525
- BRYERS JAMES D ET AL: "Local macromolecule diffusion coefficients in structurally non-uniform bacterial biofilms using fluorescence recovery after photobleaching (FRAP)" BIOTECHNOLOGY AND BIOENGINEERING, vol. 60, no. 4, 20 November 1998 (1998-11-20), pages 462-473, XP009055809 ISSN: 0006-3592
- SABANAYAGAM CHANDRAN R ET AL: "High-throughput scanning confocal microscope for single molecule analysis" APPLIED PHYSICS LETTERS, AIP, AMERICAN INSTITUTE OF PHYSICS, MELVILLE, NY, US, vol. 84, no. 7, 16 February 2004 (2004-02-16), pages 1216-1218, XP012062137 ISSN: 0003-6951
- NAGY I Z ET AL: "AN AUTOMATED METHOD FOR MEASURING LATERAL MOBILITY OF PROTEINS IN THE PLASMA MEMBRANE OF CELLS IN COMPACT TISSUES BY MEANS OF FLUORESCENCE RECOVERY AFTER PHOTO BLEACHING" MIKROSKOPIE, vol. 41, no. 1-2, 1984, pages 12-25, XP009062277 ISSN: 0026-3702
- BALINT E ET AL: "CYTOSKELETAL MODULATION OF PLASMA MEMBRANE EVENTS INDUCED BY INTERFERON-ALPHA" JOURNAL OF INTERFERON RESEARCH, vol. 12, no. 4, 1992, pages 249-255, XP009062250 ISSN: 0197-8357
- HASLER C M ET AL: "TPA-INDUCED INHIBITION OF GAP JUNCTIONAL INTERCELLULAR COMMUNICATION IS NOT MEDIATED THROUGH FREE RADICALS" TOXICOLOGY AND APPLIED PHARMACOLOGY, vol. 103, no. 3, 1990, pages 389-398, XP009062280 ISSN: 0041-008X
- HA T ET AL: "Strategy for room temperature spectroscopy of single molecules" BIOIMAGING IOP PUBLISHING UK, vol. 5, no. 3, September 1997 (1997-09), pages 99-104, XP002369165 ISSN: 0966-9051
- LOSCHENOV VICTOR B ET AL: "Spectroscopy analysis of tissues in vivo" PROC SPIE INT SOC OPT ENG; PROCEEDINGS OF SPIE - THE INTERNATIONAL SOCIETY FOR OPTICAL ENGINEERING 1995 SOCIETY OF PHOTO-OPTICAL INSTRUMENTATION ENGINEERS, BELLINGHAM, WA, USA, vol. 2370, 28 June 1994 (1994-06-28), pages 500-508, XP002369166
- KLONIS N ET AL: "Fluorescence photobleaching analysis for the study of cellular dynamics" EUROPEAN BIOPHYSICS JOURNAL SPRINGER-VERLAG GERMANY, vol. 31, no. 1, March 2002 (2002-03), pages 36-51, XP002369167 ISSN: 0175-7571

## Description

### Field of the invention

This invention relates to a method and apparatus for analysing samples of biological material. More particularly, it concerns the investigation of processes in samples such as cells, in a way that may not interfere with cell functioning. Such investigation may provide greater understanding of complex processes in life science and medicine, assist the evaluation of new drug candidates, and enable the detection of disease or abnormal processes.

### Background

Examination of live samples provides access to the dynamics of cellular and subcellular processes. The study of small-scale cellular processes has typically been carried out using light microscopy.

In some cases, the complexity of the processes is such that tools are required to isolate specific types of processes which can be studied in isolation, or in combination with a small number of other processes. The use of labels (in the form of fluorescent molecules in fluorescence microscopy) has provided tools of this nature.

A label may be extrinsic (that is, added to a sample) or intrinsic (such as a molecule which is present in the sample that is inherently distinctively coloured or fluorescent).

A fluorescent molecule has a specific excitation spectrum, being more strongly excited at some wavelengths and less strongly excited at others. It also has a specific emission spectrum, emitting more intensely at some wavelengths, and less intensely at others. The excitation and emission spectra may range from the ultraviolet to the infrared.

A wide range of fluorescent labels have been developed from chemical molecules, such as Rhodamine and Fluoroscein. Further fluorescent labels have been developed from molecules found in luminescent organisms, for example the *Aequorea* Jellyfish which has provided the Green Fluorescent Protein (GFP), and various corals providing DsRed and HcRed. These have been termed AFPs (Aequorea Fluorescent Proteins).

The fluorescent labels may be associated with specific molecules of interest (DNA, RNA, proteins, carbohydrates, antibodies, etc). Alternatively they may be made to be sensitive to certain characteristics (such as ionic concentration, pH, voltage potential, temperature, the presence of a specific enzyme, the presence of specific enzyme substrates, force), altering their fluorescent properties according to these characteristics. These labels may be introduced into cells by passing through the cell membrane or by injection. Alternatively they may be formed internally as part of the normal functioning of the cell, in the case of the genetically encoded labels such as the AFPs.

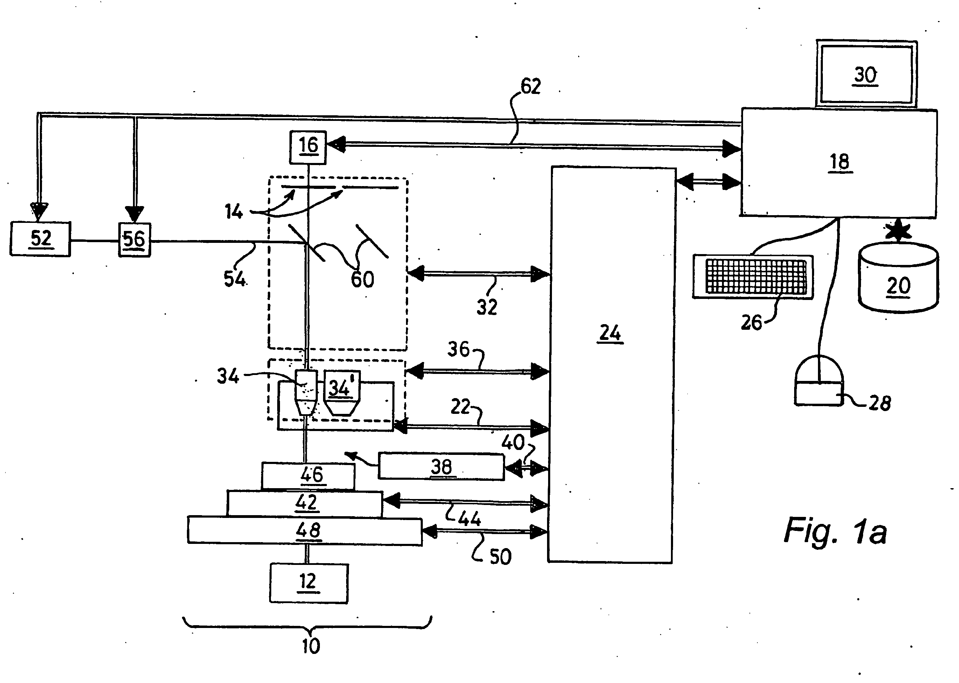

In a known apparatus for imaging samples as shown in Figure 1a, a microscope 10 is fitted with a light source 12 for illuminating a sample 4b. It may be in the form of a lamp, laser or light emitting diode, for example. The microscope is fitted with optical filters 14 so that the light emanating from the sample may be observed at selected wavebands. Examination of the spatial and temporal distribution of light from the sample may provide information on the structure and dynamics of the sample.

Phase contrast illumination may be employed to enhance imaging of thin samples. Polarised light may be used to permit the visualisation of very thin samples with small refractive index changes, for example using differential interference contrast (DIC) techniques.

The microscope is fitted with an image acquisition system, comprising a light sensitive detector 16 (sensitive from the ultraviolet to the infrared) such as a CCD camera, and recording means such as a video recorder or computer 18 with a memory device 20, so that dynamic behaviour of the sample may be captured and analysed offline. For example, the velocity, distance travelled and path of moving parts of the sample may be monitored.

The system may employ a focus drive mechanism for altering the position of the imaging focus plane. Various algorithms may be used to establish and maintain the optical focal plane. Volumetric (XYZ) and volumetric time series (XYZT) data may be acquired using deconvolution techniques. By selecting suitable excitation wavebands and/or selecting suitable optical filter sets, volumetric multi-wavelength (XYWZ) and volumetric multi-wavelength time series (XYWZT) data may be acquired.

Operations of the microscope are controlled by a microscope controller 24 in response to commands from processing means in the form of computer 18. Instructions may be entered into the computer by a user using keyboard 26, a mouse 28 and a display 30.

By way of example, Figure 1a shows control lines extending from the controller to the microscope for aspects such as filter selection along line 32, selection of an objective lens 34, 34' along line 36, focus along line 22, a liquid dispenser 38 along line 40, an environmental unit 42 along line 44 for modifying environmental conditions of a sample 46, and a sample transport unit 48 for moving the sample 46 relative to a lens of the microscope by control via line 50.

The apparatus may also include an activation light source 52 for generating an activating light beam 54 for use as discussed below. The direction of the activating light beam may be adjusted by a guide 56.

The activating light beam 54 is incident on a dichroic mirror 60. The mirror directs the beam towards an objective lens 34, and the beam is then incident on the sample 46. Light emanating from the sample 46 passes back through the objective lens 34, but is not diverted by mirror 60 so that is passes through a filter 14 before impinging on detector 16. An output signal from the detector 16 is fed to the computer 18 along line 62.

The environmental unit 42 may control the temperature of the sample, and/or the composition and flow of gas over the sample, for example.

Software loaded onto the computer enables it to acquire image data carried on the output signal from the detector.

The software also allows the user to select parameters for the operation of various aspects of the apparatus, such as the activating light beam, for example.

The controller allows the activating light beam to be directed by the setting of one or more of the following parameters:
- the one or more regions of interest on the sample (location, size and shape)
- the wavebands
- the power level
- the time of start of activation
- the duration of activation
- the number of repeat activation cycles
- the delay between activation cycles.

The computer permits recording of the image data from the detector via a digitiser at a given data rate. The filter sets in the microscope and the waveband of the illuminating light source 12 may be controlled to permit acquisition of data sets of one or more wavelengths.

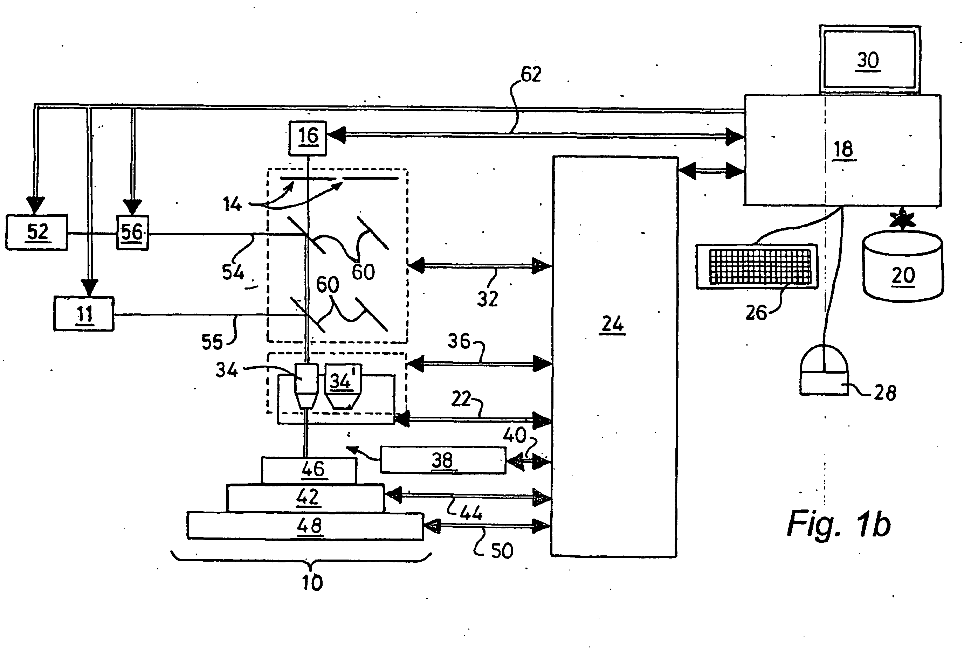

A known apparatus for imaging biological samples including fluorescent labels is shown in Figure 1b. It is similar to the apparatus shown in Figure 1a. However, instead of illumination light source 12, it includes an excitation light source 11, which generates a light beam 55. This light source is capable of exciting one or more fluorescent labels present in the sample by irradiating them with one or more specific wavebands. Filters 14 may be chosen so as to pass light emitted by labels at selected wavebands.

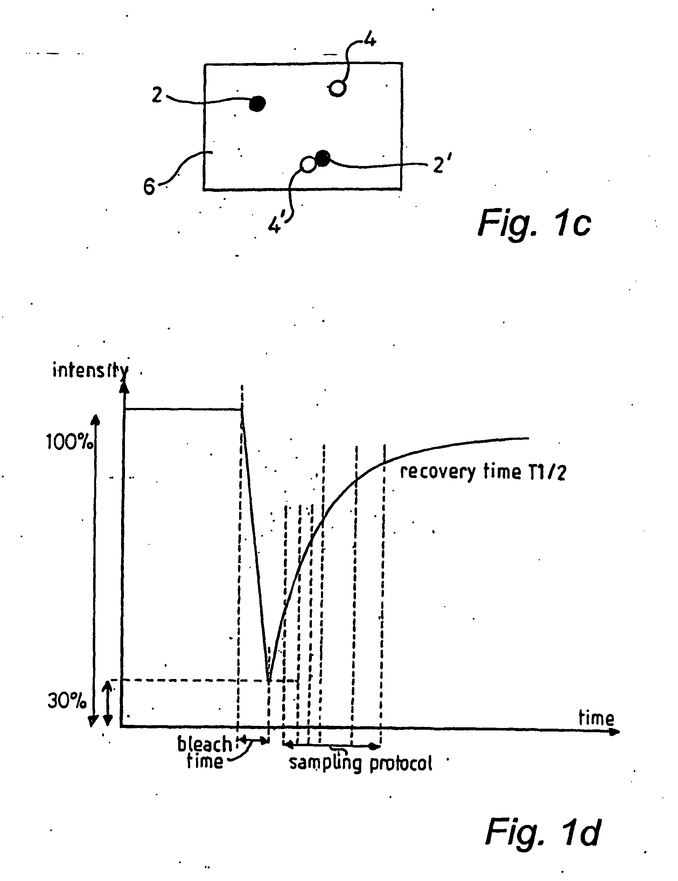

Figure 1c illustrates attachment of a fluorescent label 2, 2' to a component of interest 4, 4' in a sample 6. Multiple labels may be used to provide information on the coincident localisation of labelled components, revealing, for example, the organization of the cytoskeleton of a cell.

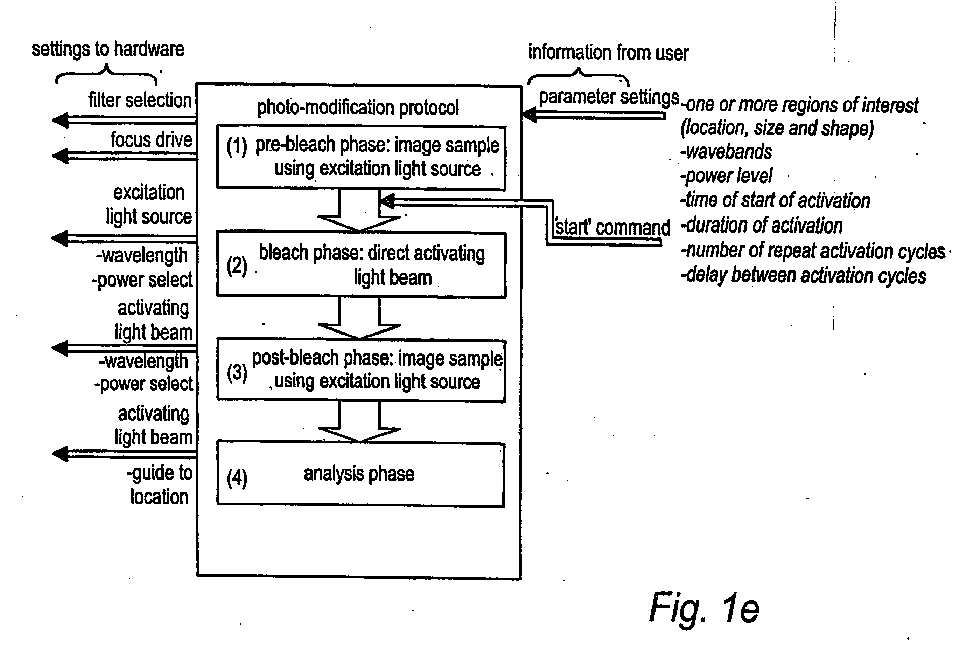

The excitation light source 11 employed in the apparatus of Figure 1 may be pulsed and the detector may be fitted with a gating device, so that the time taken for a fluorescent label to emit light following the pulse may be used to distinguish between different labels in a technique termed fluorescent lifetime imaging (FLIM) [ref. R. Cubedda, J. Phys. D: Appl. Phys., 2002, Vol. 35, R61-R76].

An activating light beam 54 may enable a deeper understanding of the dynamic processes in a sample such as a living cell (in many cases this may be based on the excitation light source) to be obtained. The activating light beam may be directed to portions of the sample in such a manner that the intense light from this beam alters the sample and/or bleaches labels present in the sample and reduces their fluorescence. By observing the subsequent changes in the light emanating from this region, and/or elsewhere in the sample, information can be obtained on mechanisms of interaction and/or exchange of various labelled components.

The beam may act to perforate a cell wall to allow the entry of an external agent, to dissect all or part of the cell, to destroy all or part of the cell, or to change the environment of the sample.

A graph plotting the intensity of fluorescence at a bleached region against time is shown in Figure 1d.

For example, the rapid recovery of fluorescence at the bleached portion suggests to what extent the label is free to return to the region. The mechanism, diffusion or local synthesis, may be determined by estimation of diffusion rates and the mobile fraction [ref. AxelRod, Biophys. J., 1977, Vol. 18, pp. 129-131.].

Further analysis and experimentation may provide access to information about molecular binding rates, assembly/disassembly and transport mechanisms [ref. J. Lippincott-Schwartz et al, Nature Supp Imaging in Cell Biol, Sept 2003, S7-S14.]. Such techniques are popular to explore the dynamics and regulation of processes in cells, for example protein trafficking, lifetime and fate (recycling and breakdown).

Conventionally, such bleaching techniques may be applied as a "bleaching protocol" comprising four phases as illustrated in Figure 1e:
(1) a pre-bleach phase in which the sample is imaged using normal fluorescence for a period of time;
(2) a bleach phase during which the activating light beam is directed to illuminate a predefined region with light of a specific wavelength with a specified higher power level for a certain period of time;
(3) a post-bleach phase in which the sample is again imaged using normal fluorescence for a period of time; and
(4) an analysis phase in which the data which has been collected is processed to determine parameters of interest, such as diffusion rate and mobile fraction according to fluorescence intensity in various regions on the image at various time points.

A set of related techniques are commonly referred to as FRAP (Fluorescence recovery after photo-bleaching). Variants of the basic protocol may be used to further explore the system, by repeated bleaching of the same region, and bleaching other portions etc (FLAP, FLIP, iFRAP) [ref. J. Lippincott-Schwartz et al, see above; Phair et al, Nature, 2000, Vol. 404, pp. 604-609.].

Alternatively, a label in the sample, the sample itself or its environment may comprise a caged compound that releases an active group when illuminated by the activating light beam - a process known as uncaging [ref. J.C. Politz, Trend Cell Biol., 1999, Vol. 9, pp. 284-287.]. The active group may be a fluorescent label. Alternatively, the active group
may not be a fluorescent label, for example the active group may instead affect the pH of the sample. The active group may have an indirectly visible or otherwise measurable effect on the sample.

In addition a technique known as pattern photo-bleaching may be using to observe changes in structure [ref. J. Ellenberg et al, Nature Supp Imaging in Cell Biol, Sept 2003, S14-S19.]. In this technique, the bleaching process is used to introduce visually distinctive landmark patterns on part of the sample, for example a grid, movement of which may be tracked over time to understand, for example, mechanisms of membrane deformation or shear.

Recent developments in fluorescent probes has resulted in new labels (photo-switchable labels) which are sensitive to the incident light, in such as way that they change their optical properties, altering their emission spectrum and/or their excitation spectrum. Examples include the photo-activatable GFP (PA-GFP) and Kaede [ref. J. Lippincott-Schwartz et al, see above.]. Furthermore, some of these probes allow their properties to be altered reversibly or irreversibly. Such probes may be activated (made to emit more intensely), or quenched (made to emit less intensely) in different wavebands. For example the kindling FP KFP1 [ref. D.M. Chudakov et al, J. Biol. Chem., 2003, Vol. 278(9), pp. 7215-7219.].

Instrumentation has been developed with an adapted activating light beam to excite photo-switchable labels and force them to undergo the change in optical properties. This may, for example, be useful in revealing or hiding labelled components during the course of an experiment and in studying long term processes such as organism development [ref. D.M. Chudakov et al, Nature Biotechnology, Feb 2003, Vol. 21, pp. 191-194.].

Hereinafter, the term photo-modification will be used to include photo-bleaching and its variants (FRAP, iFRAP, FLIP, FLAP), photo-activation, photo-switching, and pattern photo-bleaching.

Conventional control of an activating light beam involves setting up a number of activation parameters and requires the user to be able to estimate the appropriate values. In general, these are determined by trial and error using surplus material. It may be desired to set the depth of activation to be say 30% (as is the case in the illustration of Figure 1d) and the appropriate laser power setting and duration must be determined to achieve this. Similarly, the recovery time of the curve will determine the appropriate sampling regime in the post-bleach period (number of images, interval between images) which in turn is determined by the diffusion coefficient. These activation parameters must be defined prior to the experiment. They cannot be adapted to suit changing conditions. This means that the approach can only be applied to changing samples by trial and error. Thus fast changes and/or rare events can be extremely difficult to study.

In addition, in existing photo-bleaching procedures, the recovery after bleaching prevents long time course experiments (10mins+) in the tracking of change since the labels recover over shorter periods and thereafter there is no discernible bleached area.

The article "Scanning Microphotolysis: A New Photobleaching Technique based on Fast Intensity Modulation of a Scanned Laser Beam and Confocal Imaging" (Journal of Microscopy, Vol 176, Part 1, pages 23-33; 1994-10-01; Wedeking P et al) discloses with reference to its figure 1 a photobleaching method in single living cells based on fast intensity modulation of a scanned laser beam and confocal imaging. The method uses a "scamper" comprising a filter changer, an acousto-optical modulator (AOM) and a computer programmed to activate the AOM during scanning according to a freely defined image mask so that any desired pattern could be bleached ('written') into the samples and imaged ('read') at non-bleaching conditions.

The AOM together with the illumination pinhole and scanning mirror is used to write the pattern into the specimen. As shown in Fig. 1, a work station is provided, receiving input from the PMT detection means to provide a point by point image which is displaced on a computer screen. As illustrated further in Fig. 1, the point by point detected image data is also used to control the scanning mirror and via the interface shown in the "scamper" to control the irradiation conditions in respect of intensity level via the filter changer and irradiated locations via switching of the beam through the illumination pinhole under control of the AOM. The image (bleaching) mask is created using a set of preprogrammed graphic tools by importing a pre bleach image from the confocal scanning later microscope (as detected by the PMTs and registered by the workstation).

### Summary of the invention

The present invention provides a method of photo-modification of a sample of biological material including fluorescent labels using apparatus including processing means, comprising the steps of:
(a) irradiating the sample with excitation energy;
(b) detecting fluorescent radiation emitted by labels in the sample when excited by the excitation energy and outputting an output signal in response thereto;
(c) creating images of the sample according to the light detected in step (b) by processing the output signal with the processing means;
(d) analysing said images with the processing means to detect the occurrence of a predetermined event in the sample; and
(e) modifying the sample and/or labels in the sample by triggering an activating light beam in response to detection of said predetermined event in analysis step (d) by the processing means, which event is:
   - motion of a component in the sample;
   - a key event in the cell cycle and/or coincidence of more than one such event; or
   - cell death, viral invasion, endocytosis, exocytosis, tubulation, or gene transfer.

The present invention may enable combination of imaging, measurement of one or more characteristics in the images collected, and modification of the sample, and/or labels in the sample with automatic determination of the modification parameters according to the measurement made.

In comparison, conventional systems require the user to enter modification parameters manually before the start of the experiment, and so prevent dynamic setting and modification of those parameters, and thus interactive probing of complex systems.

Thus, in accordance with the invention, such modifications may be determined and executed "in real time", interactively, as the experiment progresses. This flexibility allows longer time course experiments to be conducted. It also allows rare, brief and/or rapid events to be detected and acted upon, such as all fertilisation or mutation events. Events that are difficult for a human operator to detect, such as coincident events can be detected and acted upon.

Where the present method is carried out using a light microscope, the irradiation is provided by an illumination light source. The electromagnetic radiation emanating from the sample may in that case be in the form of light transmitted by the sample if illuminated from below, or reflected light if the same is illuminated from above.

The sample includes fluorescent labels, step (a) comprises irradiating the sample with excitation energy, and step (b) comprises detecting fluorescent radiation emitted by labels in the sample when excited by the excitation energy.

The excitation energy may be in the form of electromagnetic energy (preferably light) or ionizing radiation, for example.

The modification step (e) may comprise irradiating at least a portion of the sample with an energy beam. If labels are present in the sample, this beam may be selected such that the fluorescence of labels in the portion is reduced or "bleached". In a particular embodiment, steps (a) to (e) are repeated after step (e), the period of time between the end of step (e) and the start of step (a) and/or the parameters of step (e) when repeated being dependent on said analysis by the processing means.

In a preferred embodiment, the method cycles between irradiation and modification phases, and the resultant change in the appearance of the sample is detected.

In another approach, the modification step (e) comprises irradiating at least a portion of the sample with an energy beam so as to modify the optical properties of photo-switchable fluorescent labels in the portion.

For example, it may be desirable to measure intra-cell diffusion many times over an extended period. Repeating a known FRAP experiment would have a cumulative photo-bleaching effect; soon the cell would not be visible. Using photo-switchable labels, a region can be switched rather than bleached. According to the present invention, a diffusion (or other) process may be measured, and when it is determined by the processing means that the process and/or its measurement is complete, the photo-switchable labels may be "quenched", returning them to their original state, before repeating the process. By avoiding repeated photo-bleaching a sample can be imaged for longer, and the present invention enables a process to be repeated and/or a series of measurements to be carried out in response to detected fluorescent radiation over a prolonged period.

Avoidance of repeated photo-bleaching is also beneficial as an activating light beam is often intense and its use may result in damage to the sample, or the creation of reactive species such a singlet oxygen, which may perturb the process under study, requiring sophisticated control experiments, and is thus not truly non-invasive. [Ref. D E Wolf, M Edidin, P R Dragsten, 1980, and Proc. Natl. Acad. Sci. USA 77: 2043-2045.]

After step (e), the environmental conditions of the sample may be changed and the light emanating from the sample thereafter detected for analysis.

An environmental change may be selected to cause labels in the sample to release a substance.

An environmental change can be selected to modify the manner in which the sample and/or labels in the sample respond to irradiation. For example, it may change the colour of the sample or the excitation spectrum and/or emission spectrum of labels in the sample. In particular the environmental change may be controlled activation light.

The ability to modify the response of the labels in the course of a process may avoid a need to physically introduce labels during the experiment. The procedures for introducing the labels into the sample during an experiment are quite difficult to master, requiring additional equipment, knowledge and skill on the part of the user. Experiments often have to be repeated many times. In the case of microinjection, such procedures are also invasive to the sample. It can be seen that interactive modification of labels already present in the sample may not require physical introduction of additional labels. Labels may be activated or "switched on" in a selected region.

In a further disclosed approach, the modification step may comprise modifying the physical structure of at least part of the sample. For example, this may involve forming an opening in a membrane of a selected cell in the sample to allow material to pass therethrough. The opening may be formed using a localised energy beam such as a laser, for example. In this way, an individual cell may be selected in the sample which is exhibiting behaviour of interest, and the user can form an opening in the cell wall to allow in material in response to that behaviour. The intensity of the energy beam is preferably selected such that the cell membrane is able to close or "heal" the opening shortly after its formation. Forming an opening in a cell in this way to allow the ingress of genetic material is often referred to as "transfection".

The opening may allow agents such as labels into the cell to interact with or to highlight a specific structure and/or process in the cell, or otherwise alter its optical properties. It may allow genetic material to pass into the cell. Alternatively, material may be allowed to pass into the cell so as to kill it.

In contrast to dispensing a liquid into a sample to alter the composition of the sample as a whole, modification of an individual structure such as a cell is permitted using the technique described above. It may also be employed in combination with liquid dispensing, whereby the liquid dispensing step provides the material which passes through the opening formed by the energy beam.

The environmental conditions of the sample to be changed may be its temperature, the concentration of a certain ion, the pH of the sample, the voltage potential of the sample, the presence of a specific enzyme, or the pressure of the sample. Labels in the sample may be sensitive to these conditions and activate when a predetermined range is reached.

In one example, the environment of the sample is changed by altering the composition of the gas adjacent to the sample. For example, labels in the sample may be activated by exposure to a predetermined gas or concentration of a predetermined gas, such as oxygen.

In a further example, the environment of the sample is changed by irradiating it with activation energy.

The sample may be irradiated with an activation energy beam arranged to irradiate a selected portion of the sample. The beam may define a pattern, such as a grid, for example.

A parameter (or parameters) of the activation energy irradiation determined by the processor may be one or more of the following examples: the start time of the irradiation; the portion of the sample to be irradiated; the waveband(s) of the energy; the power level; the duration of the irradiation; the number of repeat cycles of the activation irradiation energy; and the delay between repeat cycles.

The modification carried out in step (e) of a process embodying the invention may be applied to a region of the sample selected in response to the user input and/or the analysis by the processing means.

A parameter of the modification of step (e) may be determined by the processing means with reference to data stored by the apparatus.

The modification of step (e) is dependent on detection of the occurrence of a predetermined event in the sample by the processing means.

Preferably, data generated by the analysis by the processing means is stored by the apparatus so as to facilitate reference thereto in determining a parameter of a subsequent modification step.

The invention further provides apparatus for photo-modification of a sample of biological material including fluorescent labels, comprising:
- irradiation means for irradiating the sample with excitation energy;
- detection means for detecting fluorescence radiation emitted by labels in the sample when excited by the excitation energy and outputting an output signal in response thereto;
- processing means for processing the output signal from the detection means to create images of the sample;
- modification means for modifying the sample and/or labels in the sample; and
- control means for controlling the modification means so as to trigger an activating light beam in response to analysis of said images by the processing means, wherein the modification is dependent on detection of the occurrence of a predetermined event in the sample by the processing means, which event is:
- motion of a component in the sample;
- a key event in the cell cycle and/or coincidence of more than one such event; or
- cell death, viral invasion, endocytosis, exocytosis, tubulation, or gene transfer.

In a preferred embodiment, the apparatus includes data storage means for storing data generated by analysis by the processing means for reference thereto by the control means in determining a parameter of a modification by the modification means.

The apparatus may further additionally include display means for presenting to a user an indication of the electromagnetic radiation detected by the detection means, and additionally input means for enabling a user to further control at least one parameter of a modification by the modification means in response to detected radiation. Preferably, the input means enable a user to identify a specific location in a sample for a modification by the modification means.

### Detailed description of the invention

Prior art and embodiments of the invention will now be described by way of example with reference to the accompanying schematic drawings, wherein:
Figure 1a shows a block diagram of a known light microscopy apparatus for investigating processes in a sample;
Figure 1b shows a block diagram of a known fluorescence microscopy apparatus for investigating processes in a sample;
Figure 1c illustrates known use of fluorescent labels;
Figure 1d shows a typical bleach recovery curve for a fluorescent label;
Figure 1e shows a flow diagram of a known bleaching protocol; and
Figures 2 to 7 show flow diagrams of modification protocols according to respective examples of the disclosure.

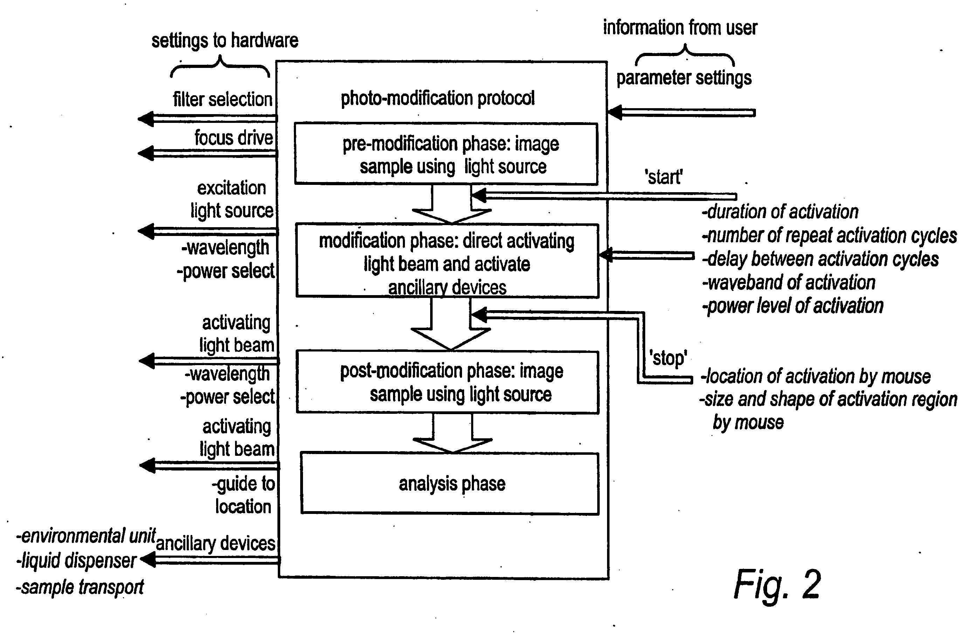

The examples described with reference to Figures 2 to 7 concern photo-modification of a sample and/or labels in a sample. It will be appreciated that the modification carried out in accordance with the invention may take other forms.

Figures 2 to 7 represent diagrammatically protocols followed by processing means of a suitable apparatus, together with associated inputs and control outputs fed to other parts of the apparatus.

Figure 2 shows a first example of the disclosure, which allows interactive control of photo-modification. A user interface allows parameters to be set and/or modified during the running of the experiment. The parameters control operation of the activating light source and/or ancillary devices such as the examples given in the Figure which may modify the sample, labels in the sample and/or the immediate environment of the sample.

In an implementation in which the activating light beam bleaches fluorescent labels in the sample, this protocol may be termed "FRAP on demand".

In the example of Figure 2, the system allows the user to set the parameters of the activating light beam comprising, the region of interest on the sample, the waveband(s), the power level at those wavebands, and the duration of activation. The user is able to image the sample and determine the start time of activation by a suitable action such as a key press.

The user may be able to determine one or more of the duration or end of activation, the number of repeat cycles and the delay between cycles of activation, and the wavelength of the activation by a suitable action such as a key press.

Furthermore, the user may be able to determine the location of activation and/or the size and shape of the irradiated region by suitable action such as manipulation of an input device such as a computer mouse.

A label may have a temperature sensitive domain, and the sample may be held on a holder, the temperature of which may be controlled by the user via the computer to activate the labels at an appropriate point in a process.

A label may be sensitive to gas such as oxygen, and the sample environmental unit controlled by the user via the computer to activate the labels in the course of an experiment.

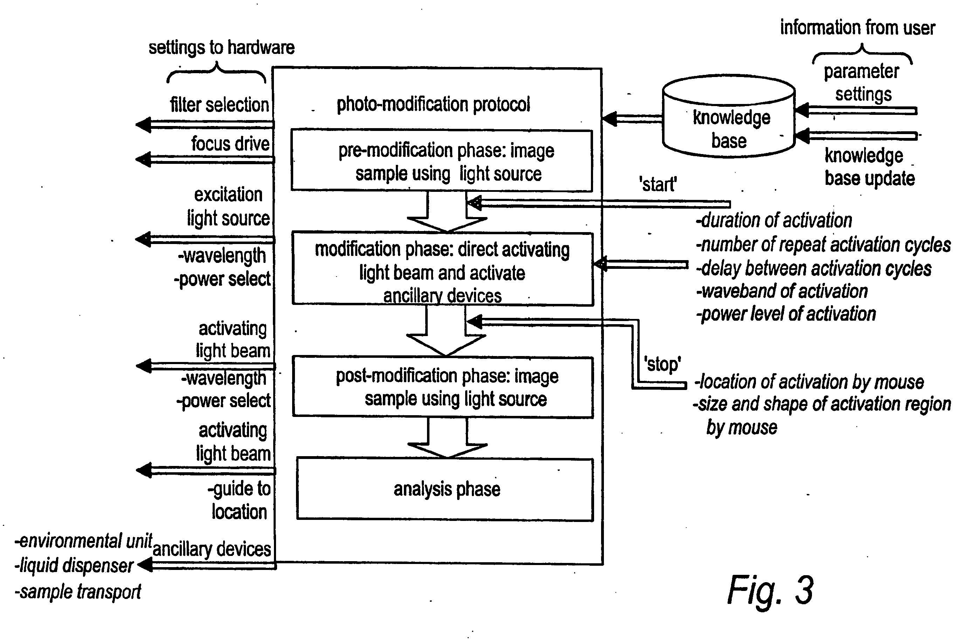

Figure 3 shows a second example that allows the use of a knowledge base to assist the user in setting and/or modifying the parameters.

In the example of Figure 3, the system is as Figure 2, and additionally allows a less experienced user to set certain high level parameters of photo-modification parameters such as the type of label to be bleached, and the system uses a knowledge base to determine the low level parameters to be used by the hardware, such as the wavelength and power level.

Additionally, a more experienced user may be able to modify the knowledge base.

In an implementation in which the activating light beam bleaches fluorescent labels in the sample, this protocol may be termed "expert FRAP".

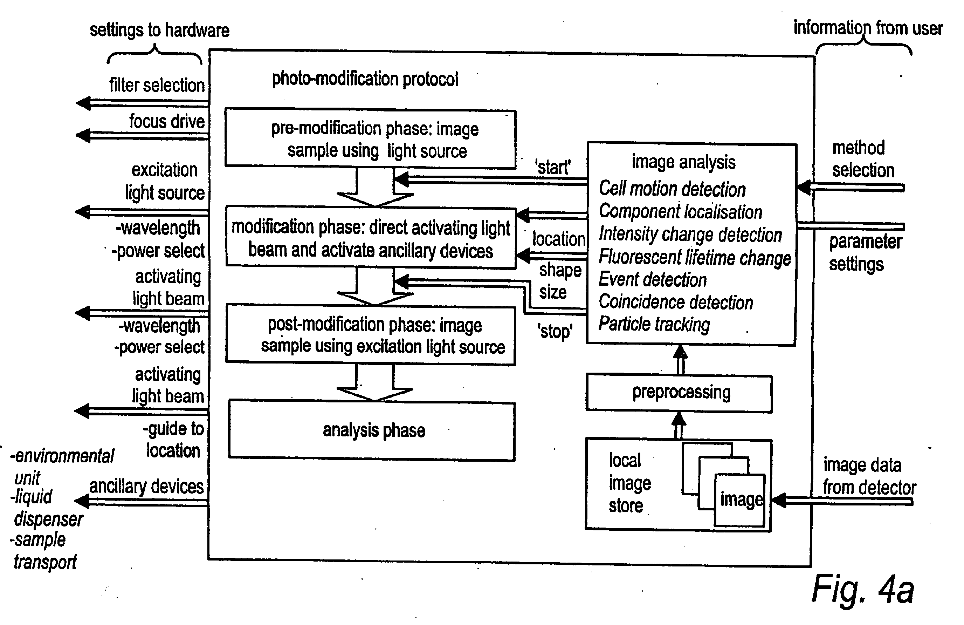

Figure 4a shows an embodiment that uses information extracted from the images collected during the pre-modification (observation) phase to set the photo-modification parameters.

In an implementation in which the activating light beam bleaches fluorescent labels in the sample, this protocol may be termed "clever FRAP".

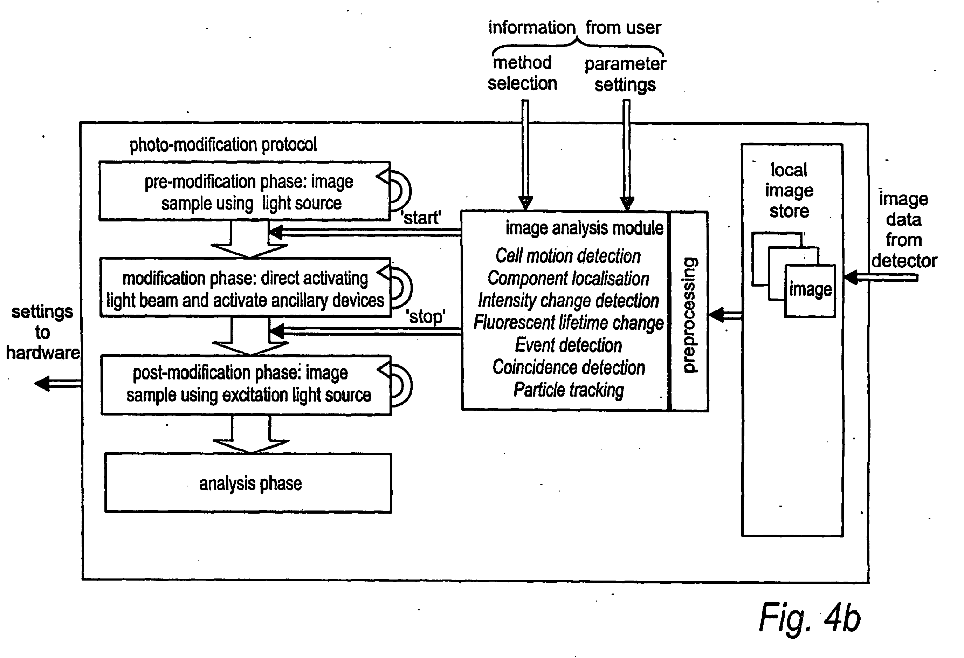

Figure 4b shows the embodiment implemented in the form of a state machine. Each phase in the protocol is repeated until an exit condition is met. For the pre-modification phase, the exit condition is when the image analysis module sends the start signal. For the modification phase, the exit condition is when the image analysis module sends the stop signal. The additional parameters to send are not shown in this diagram.

In the embodiment of Figures 4a and 4b, the system additionally allows the user to set certain parameters of the activating light beam, whilst setting others on the basis of information extracted from the data acquired during the pre-modification phase. In particular, the image data may be analysed to detect an event such as the motion of a component in the sample such as a cell in order to trigger the activating light beam.

The user may be able to additionally determine the duration or end of activation by suitable action such as a key press.

The data may be analysed either automatically according to independent claims 1, 11 or additionally by a user to:
determine location of a moving portion of the sample to direct the activating light beam;
determine a change in intensity of part of the sample to direct the activating light beam;
determine relative change in intensity of part of the sample to direct the activating light beam;
determine relative change in fluorescent lifetime of part of the sample to direct the activating light beam;
locate one or more substructures of interest, such as cellular components, organelles, vesicles or other particles, where the location and/or size of the particle(s) is used to direct the activating light beam;
locate one or more substructures of interest such as cellular components, organelles, vesicles or other particles, where the location, motion, shape, speed, direction, relative distance and/or size of the particle(s) is used to trigger the activating light beam;
detect key events in the cell cycle (such as cell division, cell death, viral invasion, endoctyosis, exocytosis, tubulation, gene transfer etc.) and/or coincidence of more than one event to trigger the activating light beam;
automatically control the instrument;
direct the setting of the irradiation source;
direct the setting of the emission wavelength. For example, by following changes in emission wavelength and adjusting the wavelength of the irradiation light to maximise the emission intensity of labels in a sample, it is possible to measure changes to characteristics of the sample and do so more effectively;
direct the setting of the polarisation of the light;
direct the setting of the focus drive mechanism and thus the plane of focus of the microscope;
direct a sample transport mechanism;
set the temperature of a sample heater; and
control a liquid dispensing mechanism.

The data may be preprocessed to remove distortions due to deformation or motion of all or part of the sample.

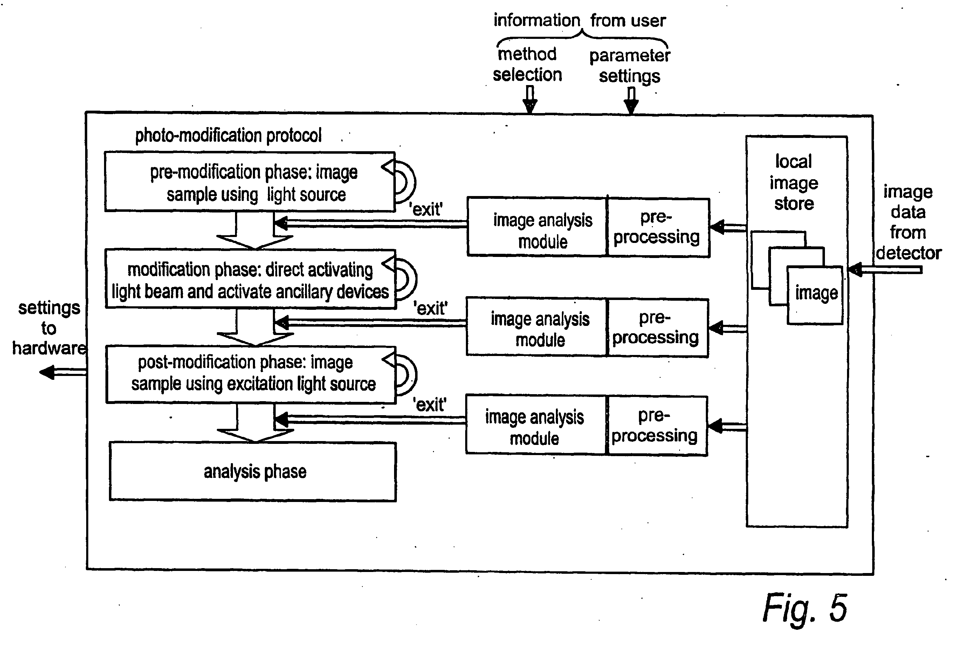

Figure 5 shows a fourth example in which an image analysis module processes the image data acquired at each phase to determine the most appropriate parameter settings, for example, the amount of light to in order to complete photo-modification.

In the example of Figure 5, the system allows the user to set certain parameters of the activating light beam, whilst the system sets others on the basis of information extracted from the data acquired during the pre-bleach phase, during the bleach phase, and during the post-bleach phase. In particular, the image data may be analysed to detect an event such as the change of a component in the sample in order to end a phase.

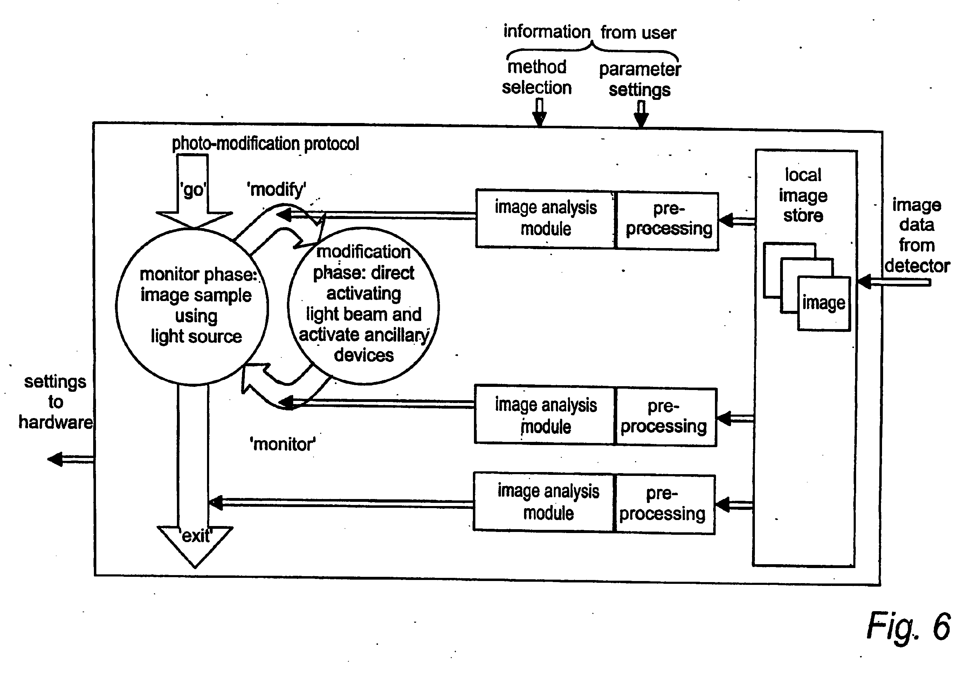

Figure 6 shows a fifth example in which the system moves from a monitor phase to a modification phase according to the information extracted from the images collected. In an implementation in which the activating light beam bleaches fluorescent labels in the sample, this protocol may be termed "automated FRAP".

This is expected to be particularly useful when used in conjunction with photo-switching labels which can be activated and quenched repeatedly and used to track the lifecycle of individual components.

In the example of Figure 6, the system allows the switching between a monitor phase during which the sample is imaged using the excitation light source, and a modification phase, during which the sample is imaged using the activating light source. The transition between the two phases and parameters for the phase is determined by the analysis of the image data collected.

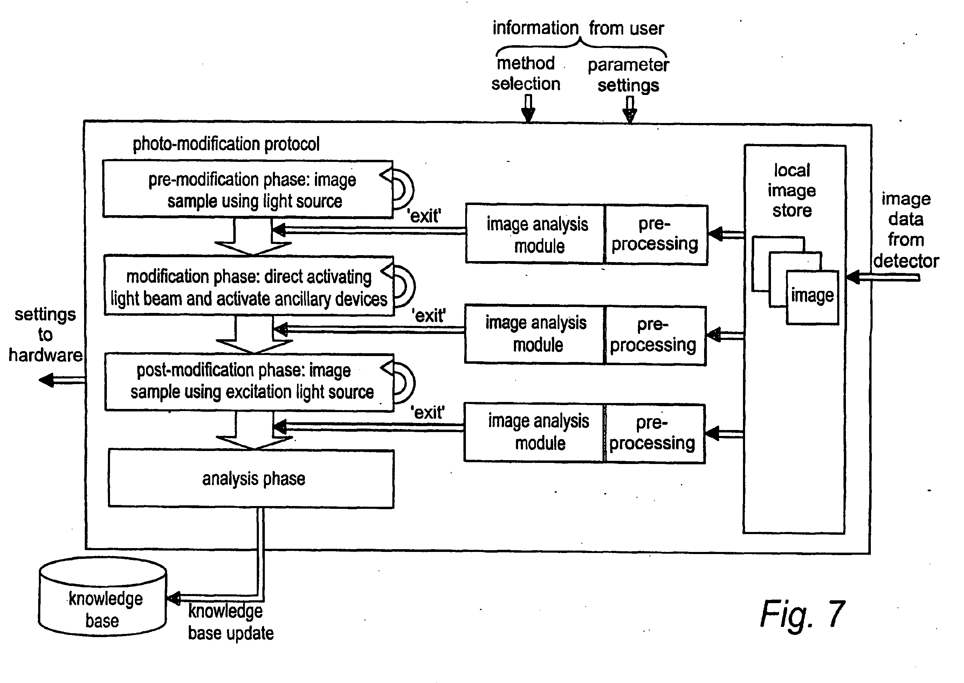

Figure 7 shows a sixth example in which the system moves from a monitor phase to a modification phase according to the information extracted from the images acquired and collects the information about the sample behaviour to the activating beam at a number of parameter settings so as to set up the knowledge base mentioned in Figure 3. In an implementation in which the activating light beam bleaches fluorescent labels in the sample, this protocol may be termed "autoconfig FRAP".

In the example of Figure 7, the system allows the control of the pre-modification phase, modification phase, post-modification phase and analysis phase as described in relation to Figure 5. The parameters used at each stage are those that permit an exploration of the possible parameters of the sample so as to quickly and efficiently determine the hardware settings (laser power, modification duration, etc) by an iterative process, and these settings are passed to knowledge base mentioned in connection with Figure 3. With the appropriate filter sets, the system may be able to image during the modification phase, for example during a bleach process, to monitor the parameters of the modification.

In a further example, the sample contains one or more photo-switchable labels, so that the activating light beam is able to activate, quench or switch the label as described above in order to observe the labelled component over short (fast moving components), medium, or long time scales (rare events or configurations).

The labels may comprise a naturally occurring fluorescent molecule such as NADH, a calcium probe such as "Fura", and/or an antibody tagged with a fluorescent molecule and bound to a selected species.

The labels may comprise a pair of fluorescent molecules with overlapping emission- excitation spectra of the types used in fluorescence resonant energy transfer ("FRET"), and the activating light beam may be set to photo-bleach the acceptor molecule in a region of the sample, so that the FRET process may be confirmed in that region.

One of the FRET pair may be a photo-switching label, and the activating light beam may be set to switch the photo-switching label in a region of the sample, so that the FRETing process may be confirmed in that region.

The labels may comprise a caged compound that releases an active group when illuminated by the activating light beam [ref. J.C. Politz, see above.].

In a further example, the excitation light source 11 may emit one or more spectral bands from a broadband light source such as a lamp. Furthermore, the excitation light source may be controllable to provide excitation light at more than one distinct waveband in order to distinguish between different labels. In some processes, it may be advantageous for the excitation light source to be pulsed.

The excitation light source may be of the type employed in fluorescent speckle microscopy [ref. M.C. Adams et al, Methods, 2002, Vol. 29, pp. 29-41.]).

In a further embodiment, the activating light beam may be derived from the same source as the excitation light.

The activating light beam may be:
pulsed;
an optical trap or optical tweezer arrangement;
a laser dissection mechanism for removing a region of interest from the sample for subsequent handling; or
a laser ablation mechanism for removing a region of interest from the sample for subsequent handling.

A localised energy beam may be employed to form an opening in a structure, such as a cell, in response to detected radiation emanating from a sample. Preferably, a laser is used. This may be a Ti:sapphire, argon-ion or frequency-shifted ND:YAG laser for example. Alternatively, a diode laser can be employed which may be less costly.

Exposing a cell to 0.3mW of violet light from a diode laser for 40ms has been found to perforate a cell membrane. The beam was focussed onto the cell using a x100 microscope objective lens to form a spot about 1 micron in diameter. This results in a power density of around 1200MW/m². The cell membrane was able to "heal" itself shortly after the process without apparently suffering any long term damage or mutation [ref. Optics Express, Vol.13, page 595].

The same light source may be used to generate the localised energy beam to form an opening in a structure as is used to generate the activating light beam, and for the excitation light beam.

In a further embodiment, the system may have one or more photo-detectors such as photo-multiplier tubes and a scanning arrangement of the excitation light beam and/or the emission light path to the detector and/or the detector to observe parts or all the sample.

The system may have more than one imaging detector fitted with different light filtering components to distinguish between different labels.

The detector may be fitted with a gating device, and the excitation light source may be pulsed, so that the time taken for the fluorescent label to emit light following the pulse may be used to distinguish between different labels.

The controller may be implemented in hardware and/or software.

In a further embodiment, the system may use a confocal principle, collecting light at a single image plane by the use of one or more pinholes and a scanning mechanism.

Alternatively, a structured illumination technique may be utilised to section a sample optically at different depths [ref. M.A.A. Neil, R. Juskaitis and T. Wilson, Optical Letters, 22(24): 1905-1907, Dec. 15 1997]. This may be more cost effective than a laser scanning confocal system, as it can be carried out using a conventional light microscope.

A total internal reflection (TIRF) principle may be employed in which the excitation light source is directed to be internally reflected at the sample-sample carrier surface, thus exciting only parts of the sample close to the surface [ref. Y. Sako and T. Yanagida, Nature Supp Imaging in Cell Biol, Sept 2003, SS1-SS5.].

Furthermore, the system may use a deconvolution principle, capturing a group of images focused at different imaging planes, and applying a deconvolution process to allow volumetric (XYZ) data to be acquired. Volumetric time series (XYZT) data, volumetric multi-wavelength (XYWZ) data, or volumetric multi-wavelength time series (XYWZT) data may be acquired.

A multi-photon microscope employing a long wavelength (800-1200nm) high speed pulsed laser may form the excitation light source and/or as the activating light beam [ref. J. Pawley, Handbook of Confocal Microscopy].

The light or fluorescence microscope may be replaced with a simplified arrangement of sample holder, magnifying objective lens and dichroic mirror to direct light from the irradiation light source and the activating light beam to the sample, and collect the emitted light beam and to direct it to a detector.

Alternatively, the fluorescence microscope may be replaced by an endoscope.

The sample may be a homogenous population of cells such as a cell culture. Alternatively, the sample may be a heterogenous multi-cellular assembly such as a cell culture; a multi-cellular assembly such as a tissue culture; a stem cell sample; a tissue sample; an organ, such as an eye or retina, or the inside of the gut or a blood vessel; a whole animal, such as the worm C.elegans, an insect, fish, mammal or amphibian; a whole or part of a plant or fungus, a dividing cell or an embryo; one of more samples held in a multi-site carrier such as a slide, or a multi-well plate, which is scanned sequentially; or a material sample undergoing some change such as diffusion of one or more species.

## Claims

1. A method of photo-modification of a sample of biological material including fluorescent labels using apparatus including processing means, comprising the steps of:
(a) irradiating the sample with excitation energy;
(b) detecting fluorescent radiation emitted by labels in the sample when excited by the excitation energy and outputting an output signal in response thereto;
(c) creating images of the sample according to the light detected in step (b) by processing the output signal with the processing means;
(d) analysing said images with the processing means to detect the occurrence of a predetermined event in the sample; and
(e) modifying the sample and/or labels in the sample by triggering an activating light beam in response to detection of said predetermined event in analysis step (d) by the processing means, which event is:
- motion of a component in the sample;
- a key event in the cell cycle and/or coincidence of more than one such event; or
- cell death, viral invasion, endocytosis, exocytosis, tubulation, or gene transfer.

2. A method of Claim 1 wherein the modification step (e) comprises irradiating a portion of the sample with the light beam such that the fluorescence of labels in the portion is reduced.

3. A method of Claim 2 wherein steps (a) to (e) are repeated after step (e), the period of time between the end of step (e) and the start of step (a) and/or the parameters of step (e) when repeated being dependent on analysis step (d).

4. A method of any preceding Claim wherein the modification step (e) comprises irradiating at least a portion of the sample with the light beam so as to modify the optical properties of photo-switchable fluorescent labels in the portion.

5. A method of any preceding Claim wherein the modification step (e) comprises modifying the physical structure of at least part of the sample.

6. A method of Claim 5 comprising forming an opening in a membrane of a selected cell in the sample to allow material to pass through the opening into the cell.

7. A method of Claim 6 wherein the opening is formed using a localised energy beam.

8. A method of any preceding Claim wherein the modification of step (e) is applied to a region of the sample selected in response to the user input and/or the analysis by the processing means.

9. A method of any preceding Claim wherein the at least one parameter of the modification of step (e) is determined by the processing means with reference to data stored by the apparatus.

10. A method of any preceding Claim wherein data generated by the analysis by the processing means is stored by the apparatus so as to facilitate reference thereto in determining a parameter of a subsequent modification step.

11. Apparatus for photo-modification of a sample (46) of biological material including fluorescent labels, comprising:
- irradiation means (11) for irradiating the sample (46) with excitation energy;
- detection means (16) for detecting fluorescence radiation emitted by labels in the sample when excited by the excitation energy and outputting an output signal in response thereto;
- processing means (18) for processing the output signal from the detection means to create images of the sample;
- modification means for modifying the sample and/or labels in the sample; and
- control means (18, 24) for controlling the modification means so as to trigger an activating light beam in response to analysis of said images by the processing means (18), wherein the modification is dependent on detection of the occurrence of a predetermined event in the sample by the processing means, which event is:
- motion of a component in the sample;
- a key event in the cell cycle and/or coincidence of more than one such event; or
- cell death, viral invasion, endocytosis, exocytosis, tubulation, or gene transfer.

12. Apparatus of Claim 11 including data storage means for storing data generated by analysis by the processing means for reference thereto by the control means in determining a parameter of a modification by the modification means.

13. Apparatus of Claim 11 or Claim 12 including display means for presenting to a user an indication of the fluorescent radiation detected by the detection means, and input means for enabling a user to control at least one parameter of a modification by the modification means in response to detected fluorescent radiation.

14. Apparatus of Claim 13 wherein the input means enable a user to identify a specific location in a sample for a modification by the modification means.

## Patentansprüche

1. Verfahren zur Photomodifizierung einer Probe biologischen Materials, das Fluoreszenzmarker enthält, unter Verwendung von Apparatur, die Verarbeitungsmittel enthält, umfassend die folgenden Schritte:
(a) Bestrahlen der Probe mit Anregungsenergie;
(b) Erfassen von Fluoreszenzstrahlung, die bei Anregung durch die Anregungsenergie von Markern in der Probe abgegeben wird, und Ausgeben eines Ausgangssignals als Antwort darauf;
(c) Erzeugen von Bildern der Probe nach dem in Schritt (b) erfassten Licht durch Verarbeiten des Ausgangssignals mit Verarbeitungsmitteln;
(d) Analysieren der Bilder mit den Verarbeitungsmitteln zur Erfassung, ob ein vorbestimmtes Ereignis in der Probe aufgetreten ist; und
(e) Modifizieren der Probe und/oder Marker in der Probe durch Auslösen eines Aktivierungslichtstrahls als Antwort auf die Erfassung des vorbestimmten Ereignisses in Analyseschritt (d) durch die Verarbeitungsmittel, wobei es sich bei dem Ereignis um:
- Bewegung einer Komponente in der Probe;
- ein Schlüsselereigniss in dem Zellzyklus und/oder Koinzidenz von mehr als einem solchen Ereignis oder
- Zelltot, Virusinvasion, Endozytose, Exozytose, Tubulierung oder Gentransfer,
handelt.

2. Verfahren nach Anspruch 1, wobei der Modifizierungsschritt (e) das Bestrahlen eines Abschnitts der Probe mit dem Lichtstrahl derart, dass die Fluoreszenz von Markern in dem Abschnitt reduziert ist, umfasst.

3. Verfahren nach Anspruch 2, wobei Schritte (a) bis (e) nach Schritt (e) wiederholt werden, wobei die Zeitdauer zwischen dem Ende von Schritt (e) und dem Anfang von Schritt (a) und/oder die Parameter von Schritt (e) bei Wiederholung von dem Analyseschritt (d) abhängt oder abhängen.

4. Verfahren nach einem vorhergehenden Anspruch, wobei der Modifizierungsschritt (e) das Bestrahlen mindestens eines Abschnitts der Probe mit dem Lichtstrahl, um die optischen Eigenschaften von photoschaltbaren Fluoreszenzmarkern in dem Abschnitt zu modifizieren, umfasst.

5. Verfahren nach einem vorhergehenden Anspruch, wobei der Modifizierungsschritt (e) das Modifizieren der physikalischen Struktur von mindestens einem Teil der Probe umfasst.

6. Verfahren nach Anspruch 5, umfassend das Bilden einer Öffnung in einer Membran einer ausgewählten Zelle in der Probe, damit Material durch die Öffnung in die Zelle tritt.

7. Verfahren nach Anspruch 6, wobei die Öffnung unter Verwendung eines lokalisierten Energiestrahls gebildet wird.

8. Verfahren nach einem vorhergehenden Anspruch, wobei der Modifizierungsschritt (e) auf einen Bereich der Probe, ausgewählt als Antwort auf die Benutzereingabe und/oder die Analyse durch die Verarbeitungsmittel, angewendet wird.

9. Verfahren nach einem vorhergehenden Anspruch, wobei der mindestens eine Parameter der Modifizierung in Schritt (e) durch die Verarbeitungsmittel mit Bezug auf die durch die Vorrichtung gespeicherten Daten bestimmt wird.

10. Verfahren nach einem vorhergehenden Anspruch, wobei durch die Analyse durch die Verarbeitungsmittel erzeugten Daten durch die Vorrichtung gespeichert werden, um den Bezug darauf bei der Bestimmung eines Parameters eines darauffolgenden Modifizierungsschritts zu erleichtern.

11. Vorrichtung zur Photomodifizierung einer Probe (46) biologischen Materials, das Fluoreszenzmarker enthält, die Folgendes umfasst:
- Bestrahlungsmittel (11) zum Bestrahlen der Probe (46) mit Anregungsenergie;
- Erfassungsmittel (16) zum Erfassen von Fluoreszenzstrahlung, die bei Anregung durch die Anregungsenergie von Markern in der Probe abgegeben wird, und Ausgeben eines Ausgangssignals als Antwort darauf;
- Verarbeitungsmittel (18) zum Verarbeiten des Ausgangssignals von den Erfassungsmitteln zur Erzeugung von Bildern von der Probe;
- Modifizierungsmittel zur Modifizierung der Probe und/oder Markern in der Probe; und
- Steuermittel (18, 24) zum Steuern der Modifizierungsmittel, um einen Aktivierungslichtstrahl als Antwort auf die Analyse der Bilder durch die Verarbeitungsmittel (18) auszulösen, wobei die Modifizierung von der Erfassung, ob ein vorbestimmtes Ereignis in der Probe aufgetreten ist, durch die Verarbeitungsmittel abhängt, wobei es sich bei dem Ereignis um:
- Bewegung einer Komponente in der Probe;
- ein Schlüsselereignis in dem Zellzyklus und/oder Koinzidenz von mehr als einem solchen Ereignis oder
- Zelltot, Virusinvasion, Endozytose, Exozytose, Tubulierung oder Gentransfer,
handelt.

12. Vorrichtung nach Anspruch 11, enthaltend Datenspeichermittel zur Speicherung von Daten, die durch die Analyse durch die Verarbeitungsmittel für den Bezug darauf durch die Steuermittel bei der Bestimmung eines Parameters einer Modifizierung erzeugt werden.

13. Vorrichtung nach Anspruch 11 oder Anspruch 12, enthaltend Display-Mittel zur Darstellung für einen Benutzer einer Anzeige der von den Erfassungsmitteln erfassten Fluoreszenzstrahlung, und Eingabemittel, damit ein Benutzer mindestens einen Parameter einer Modifizierung durch die Modifizierungsmittel als Antwort auf erfasste Fluoreszenzstrahlung steuern kann.

14. Vorrichtung nach Anspruch 13, wobei die Eingabemittel es einem Benutzer gestatten, eine spezifische Stelle in einer Probe für eine Modifizierung durch die Modifizierungsmittel zu identifizieren.

## Revendications

1. Procédé de photomodification d'un échantillon de matière biologique contenant des marqueurs fluorescents avec un appareil comportant des moyens de traitement, comprenant les étapes consistant à :
(a) irradier l'échantillon avec une énergie d'excitation ;
(b) détecter le rayonnement fluorescent émis par des marqueurs dans l'échantillon lorsqu'ils sont excités par l'énergie d'excitation et émettre un signal de sortie en réponse à celui-ci ;
(c) créer des images de l'échantillon en fonction de la lumière détectée à l'étape (b) en traitant le signal de sortie avec les moyens de traitement ;
(d) analyser lesdites images avec les moyens de traitement pour détecter l'occurrence d'un événement prédéterminé dans l'échantillon ; et
(e) modifier l'échantillon et/ou des marqueurs dans l'échantillon en déclenchant un faisceau lumineux d'activation en réponse à la détection dudit événement prédéterminé à l'étape d'analyse (d) par les moyens de traitement, lequel événement est :
- un mouvement d'un composant dans l'échantillon ;
- un évènement essentiel dans le cycle cellulaire et/ou la coïncidence de plusieurs tels événements ; ou
- la mort de cellules, une invasion virale, une endocytose, une exocytose, une tubulation, ou un transfert de gènes.

2. Procédé selon la revendication 1 dans lequel l'étape de modification (e) comprend l'irradiation d'une partie de l'échantillon avec le faisceau lumineux de telle sorte que la fluorescence de marqueurs dans la partie est réduite.

3. Procédé selon la revendication 2 dans lequel les étapes (a) à (e) sont répétées après l'étape (e), la période de temps entre la fin de l'étape (e) et le début de l'étape (a) et/ou les paramètres de l'étape (e) lorsqu'elle est répétée dépendant de l'étape d'analyse (d).

4. Procédé selon l'une quelconque des revendications précédentes dans lequel l'étape de modification (e) comprend l'irradiation d'au moins une partie de l'échantillon avec le faisceau lumineux de manière à modifier les propriétés optiques de marqueurs fluorescents photocommutables dans la partie.

5. Procédé selon l'une quelconque des revendications précédentes dans lequel l'étape de modification (e) comprend la modification de la structure physique d'au moins une partie de l'échantillon.

6. Procédé selon la revendication 5 comprenant la formation d'une ouverture dans une membrane d'une cellule sélectionnée dans l'échantillon pour permettre à la matière de passer à travers l'ouverture dans la cellule.

7. Procédé selon la revendication 6 dans lequel l'ouverture est formée en utilisant un faisceau d'énergie localisée.

8. Procédé selon l'une quelconque des revendications précédentes dans lequel la modification de l'étape (e) est appliquée à une région de l'échantillon sélectionnée en réponse à une saisie de l'utilisateur et/ou à l'analyse par les moyens de traitement.

9. Procédé selon l'une quelconque des revendications précédentes dans lequel l'au moins un paramètre de la modification de l'étape (e) est déterminé par les moyens de traitement en référence à des données stockées par l'appareil.

10. Procédé selon l'une quelconque des revendications précédentes dans lequel les données générées par l'analyse par les moyens de traitement sont stockées par l'appareil de manière à faciliter la référence à celles-ci lors de la détermination d'un paramètre d'une étape de modification consécutive.

11. Appareil pour la photomodification d'un échantillon (46) de matière biologique contenant des marqueurs fluorescents, comportant :
- des moyens d'irradiation (11) pour irradier l'échantillon (46) avec une énergie d'excitation ;
- des moyens de détection (16) pour détecter le rayonnement de fluorescence émis par des marqueurs dans l'échantillon lorsqu'ils sont excités par l'énergie d'excitation et émettre un signal de sortie en réponse à celui-ci ;
- des moyens de traitement (18) pour traiter le signal de sortie des moyens de détection afin de créer des images de l'échantillon ;
- des moyens de modification pour modifier l'échantillon et/ou des marqueurs dans l'échantillon ;
et
- des moyens de commande (18, 24) pour contrôler les moyens de modification de manière à déclencher un faisceau lumineux d'activation en réponse à l'analyse desdites images par les moyens de traitement (18), la modification dépendant de la détection de l'occurrence d'un événement prédéterminé dans l'échantillon par les moyens de traitement, lequel événement est :
- un mouvement d'un composant dans l'échantillon ;
- un évènement essentiel dans le cycle cellulaire et/ou la coïncidence de plusieurs tels événements ; ou
- la mort de cellules, une invasion virale, une endocytose, une exocytose, une tubulation, ou un transfert de gènes.

12. Appareil selon la revendication 11 comportant des moyens de stockage de données pour stocker les données générées par l'analyse par les moyens de traitement pour référence à celles-ci par les moyens de commande lors de la détermination d'un paramètre d'une modification par les moyens de modification.

13. Appareil selon la revendication 11 ou la revendication 12 comportant des moyens d'affichage pour présenter à un utilisateur une indication du rayonnement fluorescent détecté par les moyens de détection, et des moyens de saisie pour permettre à un utilisateur de contrôler au moins un paramètre d'une modification par les moyens de modification en réponse au rayonnement fluorescent détecté.

14. Appareil selon la revendication 13 dans lequel les moyens de saisie permettent à un utilisateur d'identifier un emplacement spécifique dans un échantillon pour une modification par les moyens de modification.
